(19) Europäisches Patentamt
European Patent Office
Office européen des brevets

(11) **EP 4 268 719 A1**

(12) **EUROPEAN PATENT APPLICATION**

(43) Date of publication:
**01.11.2023 Bulletin 2023/44**

(21) Application number: **23170041.0**

(22) Date of filing: **26.04.2023**

(51) International Patent Classification (IPC):
**A61B 5/145** *(2006.01)*     **A61B 5/00** *(2006.01)*
**A61M 5/172** *(2006.01)*

(52) Cooperative Patent Classification (CPC):
**A61B 5/14532; A61B 5/4839; A61B 5/6833;
A61B 5/6843; A61M 5/1723;** A61B 2560/0247

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(30) Priority: **26.04.2022 US 202263334808 P**

(71) Applicant: **Insulet Corporation
Acton MA 01720 (US)**

(72) Inventor: **NARAYANASWAMI, Rangarajan
Weston (US)**

(74) Representative: **Peterreins Schley
Patent- und Rechtsanwälte PartG mbB
Hermann-Sack-Straße 3
80331 München (DE)**

(54) **COMPRESSION DETECTION AND COMPENSATION FOR A BIOLOGICAL SENSOR SECURED TO A USER**

(57)     The exemplary embodiments may detect compression of a biological sensor and/or biological tissue of the user and may prompt or take corrective action to address the compression. A compression sensor may be provided to sense compression of the biological sensor and/or biological tissue of the user . Sensor data from the compression sensor may be processed to detect the compression. The detection of the compression may trigger corrective action. The corrective action may include an alert to alert the user to suggest a change in position or body orientation so as to alleviate the compression. The corrective action may include adjusting the biological sensor data to compensate for the compression or may include adjusting the quantity or rate of medicament delivered to a user to compensate for the compression. In some exemplary embodiments a data logger is provided to work with the biological sensor. The data logger may automatically log compression sensor data and biological sensor data.

Figure 1

EP 4 268 719 A1

## Description

## BACKGROUND

Biological sensors, such as glucose sensors, may be attached to users to detect biological information from a user on an ongoing basis. Such biological sensors may be unintentionally compressed by the users. For example, when a user is sleeping, the weight of the user's body may rest on top of the biological sensor and cause compression of the biological sensor and/or underlying body tissue. Similarly, when the user is awake but sitting or lying down, the user may unintentionally compress the biological sensor and/or underlying body tissue.

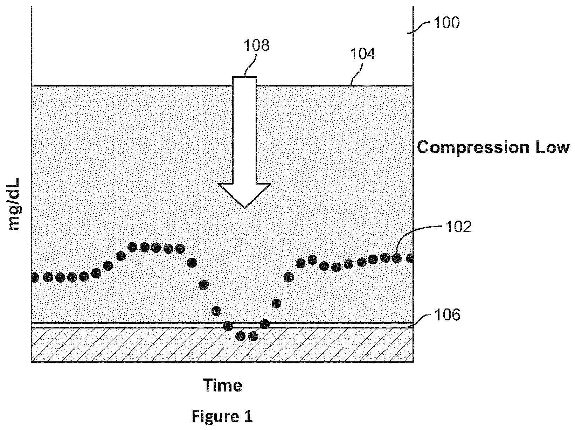

The compression by the user may affect operation of the biological sensor. Consider the case where the biological sensor is a glucose monitor that monitors the glucose level of the user. Compression of the glucose monitor or body tissue may cause a non-negligible drop in the glucose level detected by the glucose monitor. Figure 1 depicts an illustrative plot 100 of glucose level in mg/dL as detected by the glucose monitor over time. The dots 102 shown on the plot 100 represent discrete glucose levels sensed by the glucose monitor at periodic intervals, such as every 5 minutes. A high threshold 104 and a low threshold 106 are shown to define the bounds of a desired glucose range. As can be seen and as referenced by arrow 108, there is a discernable trough in the glucose levels. This trough is the result of compression of the glucose monitor or underlying body tissue by a user. The problem posed by the trough is that the sensed glucose levels that form the trough are artificially decreased and not accurate. The compression decreases the interstitial space where the detecting element for the glucose monitor is positioned and can result in less glucose per unit of volume at the detecting element or an otherwise erroneous sensor reading.

## SUMMARY

In accordance with a first inventive facet, an electronic device which is attached to a user is provided. The electronic device includes a medicament delivery unit for delivering medicament to the user and a biological sensor for sensing one or more analytes or biological data of the user and generating biological sensor data indicative of the sensed biological data. The electronic device further includes a compression sensor for sensing compression of the biological sensor and/or biological tissue of the user and generating compression sensor data. One or more housings encase the medicament delivery unit and the biological sensor, in particular the analyte level sensor. The electronic device additionally includes a processor configured for receiving the compression sensor data from the compression sensor and taking corrective action when the compression sensor data from the compression sensor indicates excessive compression of the biological sensor and/or biological tissue of the user.

The corrective action may include triggering an alarm that indicates to the user that there is excessive compression of the biological sensor. The alarm may be part of the electronic device and the alarm may include at least one of a vibratory alarm, an audio alarm, a visual alarm, a message transmitted to the user, and/or an indication on a user interface of a device used by the user (e.g., via a smartphone app that is associated with the biological sensor). The corrective action may include one of modifying an amount of medicament delivered to the user by the medicament delivery unit to compensate for the excessive compression or adjusting/correcting the biological sensor data from the biological sensor to compensate for the excessive compression. The one or more housings may be a single housing. In some embodiments, the medicament delivery unit and the biological sensor may be encased by a single housing. In some embodiments, the electronic device may include a compliant layer to which the compression sensor is secured or positioned against, and the compliant layer may be secured to the one or more housings, in particular the single housing. The electronic device also may include an additional compliant layer positioned so that the compression sensor is situated between the compliant layer and the additional compliant layer. The electronic device may further include an adhesive layer having an adhesive for attaching the electronic device to the user.

In accordance with another inventive facet, a glucose monitor is provided. The glucose monitor includes a glucose sensor for sensing a glucose level of a user and generating glucose level readings indicative of the sensed glucose level. The glucose monitor includes a compression sensor for sensing amounts of compression of the glucose monitor by the user over time and/or biological tissue of the user and for generating compression sensor readings indicative of the sensed amounts of compression. The glucose monitor further includes a memory for storing the glucose level readings and the compression sensor readings and includes a wireless transmitter for sending the glucose level readings and the compression sensor readings in the storage to an external electronic device. The glucose monitor additionally includes a processor configured for storing the glucose level readings and the compression sensor readings in the memory and for forwarding the glucose level readings from the memory to the wireless transmitter for transmission to the external electronic device.

The processor may be further configured to forward at least some of the compression sensor readings from the memory to the wireless transmitter for transmission to the external electronic device. The external electronic device may be one of an insulin delivery device or a management device for the glucose sensor and/or the insulin delivery device, and the processor may be further configured to cause the wireless transmitter to transmit at least some of the glucose level readings and compression sensor readings from the memory to the insulin delivery device or the management device for the insulin delivery device. The glucose monitor may include one or more alarms, such as an auditory alarm and/or a vibratory alarm, for warning the user of excessive compression of the glucose monitor and/or biological tissue of the user. The processor may be further configured to receive an indication that there is excessive compression of the glucose monitor and/or biological tissue of the user from the external device and to trigger the one or more alarms responsive to receiving the indication.

In accordance with a further inventive facet, a method is performed by a processor of an electronic device. The method includes receiving sensor data from a compression sensor secured to or positioned against a glucose monitor for a user and processing the sensor data from the compression sensor with the processor to determine whether the glucose monitor and/or biological tissue of the user is being compressed. If compression is detected, corrective action is triggered with the processor responsive to the determining that the glucose monitor and/or biological tissue of the user is being compressed.

The compression sensor may be an electronic sensor. The compression sensor may be a mechanical sensor. The compression sensor may be secured to a bottom surface of the glucose monitor that faces the user. The corrective action may include initiating one or more alarms or messages to alert the user of the glucose monitor and/or biological tissue of the user being compressed. The corrective action may include adjusting/correcting glucose readings from the glucose monitor to compensate for the compression. The corrective action may include advising an insulin delivery device or a management device of the insulin delivery device of the excessive compression of the glucose monitor and/or the biological tissue of the user. The electronic device may be an insulin delivery device configured to be secured to the user or a management device for the insulin delivery device configured to be secured to the user. Other corrective action may be a message output to the user to indicate that compression and/or depressed glucose readings have been detected.

**BRIEF DESCRIPTION OF THE DRAWINGS**

Figure 1 depicts an illustrative plot of glucose level values for a user with a trough due to compression of a conventional glucose sensor.

Figure 2A depicts a diagram illustrating communication paths among components of an automatic medicament delivery environment in exemplary embodiments.

Figure 2B depicts a flowchart of illustrative steps that may be performed in exemplary embodiments to detect excessive compression of a biological sensor and taking corrective action in response to the detection of the excessive compression.

Figure 3 depicts a diagram of illustrative types of corrective actions that may be taken in exemplary embodiments.

Figure 4 depicts a diagram of illustrative types of alerts that may be used in exemplary embodiments.

Figure 5 depicts a flowchart of illustrative steps that may be performed in exemplary embodiments to adjust a biological sensor value to compensate for compression of the biological sensor.

Figure 6 depicts a flowchart of illustrative steps that may be performed in exemplary embodiments to adjust medicament deliveries by a medicament delivery device or unit to compensate for compression of the biological sensor.

Figure 7A depicts a block diagram of components of a data logger in exemplary embodiments.

Figure 7B depicts types of sensor data stored in the storage of the data logger in exemplary embodiments.

Figure 8 depicts components of a data logger and wireless communication subsystem in exemplary embodiments.

Figure 9 depicts three different views of an illustrative data logger for exemplary embodiments.

Figure 10 depicts a flowchart of illustrative steps that may be performed by the data logger in exemplary embodiments to log and forward sensor data.

Figure 11 depicts a flowchart of illustrative steps that may be performed by the data logger in exemplary embodiments to provide swap protection to avoid data loss during the swapping of medicament delivery devices.

Figure 12A depicts an example of a co-located insulin and CGM system of exemplary embodiments.

Figure 12B depicts an example of a co-located insulin and CGM system of exemplary embodiments having a voltage source for a force sensitive resistor.

Figure 13 depicts components of an illustrative medicament delivery device suitable for exemplary embodiments.

## DETAILED DESCRIPTION

The exemplary embodiments may detect compression of a biological sensor and/or underlying body tissue (which may generally be referred to herein as compression of a biological sensor) and may prompt or take corrective action to address excessive compression. A compression sensor may be provided to sense compression of the biological sensor. Sensor data from the compression sensor may be processed to detect excessive compression. The excessive compression may be defined by a compression threshold. For example, the compression sensor data may be numerical data values and the compression threshold may be a specific numerical data value. Accordingly, in some embodiments, the sensor data from the compression sensor may indicate excessive compression, when the compression sensor data exceeds the compression threshold. The compression threshold may depend on the severity of the influence of the compression on the detecting element. In some embodiments, the compression threshold may relate to an (expected) measurement deviation of the biological sensor of at least 2%, more specifically 5%, even more specifically at least 10% and in particular at least 20%. The expected measurement deviation may be determined for a specific type of biological sensor. In some embodiments, the compression threshold may relate to an (expected) measurement deviation of the biological sensor of between about 2% to about 60%, more specifically between about 5% to about 50%, even more specifically between about 10% to about 45% and in particular between about 20% to about 40%. The expected measurement deviation may be based on theoretical models or empirical data. The detection of the excessive compression may trigger corrective action. In some embodiments, the detection of a compression above the compression threshold may trigger the corrective action. In some embodiments, the compression sensor 204 generating compression data, indicating that the compression threshold is exceeded, may trigger the corrective action. The corrective action may include an alert to cause the user to change position or body orientation so as to alleviate the excessive compression. The corrective action may include adjusting/correcting the biological sensor data to compensate for the compression or may include adjusting/correcting the quantity or rate of medicament delivered to a user to compensate for the excessive compression. It should be noted, that the compression threshold may not be a constant value, but may be varied throughout the day.

In some exemplary embodiments a data logger is provided to work with the biological sensor. The data logger automatically logs compression sensor data and biological sensor data. The data logger may wirelessly communicate with a medicament delivery device or a management device for the biological sensors and/or the medicament delivery device. The data logger may wirelessly transmit the compression sensor data and/or the biological sensor data to the medicament delivery device or to the management device for processing, including detecting the excessive compression and triggering corrective action. In some exemplary embodiments, the data logger, instead, may process the compression sensor data. Alarms may be provided in the data logger to alert the user of the compression.

The data logger may be useful when a disposable medicament delivery device is being swapped out. For example, certain insulin pumps require replacement with a new insulin pump every few days. Conventionally, during the swap out, biological sensor data may be lost until the new medicament delivery device is secured to the user and operating normally. With the data logger, the biological sensor data may be logged with the logger during the swap out and communicated from the logger to the medicament delivery device or the management device for the medicament delivery device. Thus, the data logger may prevent the biological sensor data from being lost.

The discussion below focuses on instances where the biological sensor is an analyte level sensor like a glucose and/or ketone sensor that senses glucose and/or ketone levels of the user and the medicament delivery device is a device that delivers insulin, GLP-1, pramlintide, or co-formulations of insulin, GLP-1, and/or pramlintide, glucagon, or other liquid drugs. In particular, in some embodiments, the biological sensor may be configured to detect a user's glucose level. The glucose level detected by the biological sensor may also be referred to as glucose level data or glucose reading. In some embodiments, the biological sensor may be configured to measure the glucose concentration in interstitial fluid. In some embodiments, the biological sensor may comprise a detecting element configured to be disposed in biological tissue during use, in particular biological tissue of the user, more specifically wherein the detecting element is configured to be disposed in contact with interstitial fluid during, or within the interstitial space during use. Accordingly, in some embodiments the biological data may comprise the user's glucose levels, in particular the user's blood glucose level, and/or the user's ketone levels, in particular the user's blood ketone level. The biological sensor senses biological data relating to the user. The biological sensor, in some embodiments, may be, for example, a blood pressure monitor, a heartbeat monitor, a temperature sensor, a blood oxygen sensor, a perspiration sensor, a breathing rate sensor, or the like. The biological sensor may be an analyte level sensor that measures one or more analyte levels of the user. Accordingly, in some embodiments the biological data may comprise blood pressure value, a heartrate, a temperature, a blood oxygen level, a perspiration rate and/or a breathing rate.

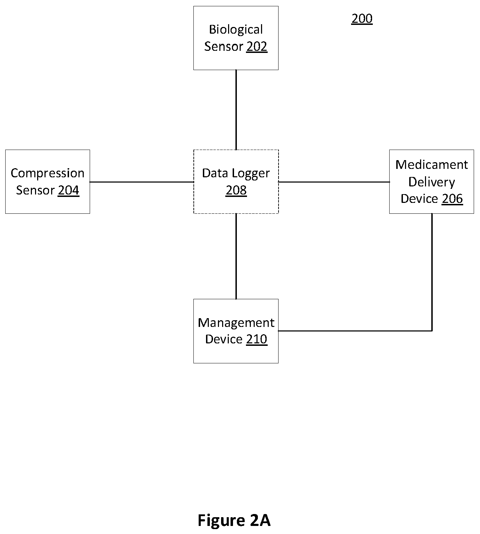

Figure 2A depicts a block diagram 200 of components of a medicament delivery system that may be used in exemplary embodiments. A biological sensor 202 may be secured to a user to detect a biological characteristic of the user, such as glucose level, ketone level, heartbeat, temperature, blood oxygen level or the like. In some embodiments, the biological sensor may be secured to the user, such as by having an adhesive layer that holds the sensor in place secured to the user. In some exemplary embodiments, the biological sensor is a continuous glucose monitor (CGM). A compression sensor 204 is provided to sense compression of the biological sensor 202. The compression sensor 204 may be a pressure sensor that senses pressure, a force sensor that senses magnitude of force and possible direction of force, a strain gauge that senses strain, a rigid compliant capacitive sensor that senses an external mechanical force, or the like. The compression sensor 204 may be an electrical sensor or a mechanical sensor. In some embodiments, the compression sensor 204 may be a configured to sense force, pressure, strain and/or mechanical stress. In some embodiments, the compression sensor 204 may be a force-sensing resistor or force-sensing capacitor. In some embodiments, the compression sensor may comprise a force-sensing layer. The excessive compression may be defined by a force threshold. The term "force threshold" herein may relate to a specific value of force, pressure, strain or mechanical stress. Accordingly, in some embodiments, the compression sensor may indicate excessive compression, when the compression sensor data exceeds a force, pressure, strain or mechanical stress defined by a force threshold. When the compression sensor data shows that the compression exceeds the force threshold, this may be defined as excessive compression. In some embodiments, the compression sensor 204 generating compression data, indicating that the force threshold is exceeded, may trigger the corrective action. In some embodiments, the force threshold may relate to an (expected) measurement deviation of the biological sensor of at least 2%, more specifically 5%, even more specifically at least 10% and in particular at least 20%. In some embodiments, the force threshold may relate to an (expected) measurement deviation of the biological sensor of between about 2% to about 60%, more specifically between about 5% to about 50%, even more specifically between about 10% to about 45% and in particular between about 20% to about 40%. In some embodiments, the pressure threshold may relate to an (expected) measurement deviation of the biological sensor of between about 2% to about 60%, more specifically between about 5% to about 50%, even more specifically between about 10% to about 45% and in particular between about 20% to about 40%. In some embodiments, the force threshold may relate to a force of at least 1 Newton, more specifically at least 3 Newton and in particular at least 10 Newton. In some embodiments, the force threshold may relate to a force of between about 1 Newton to about 500 Newton, more specifically between about 3 Newton to about 100 Newton and in particular between about 10 Newton to about 30 Newton. In some embodiments, the force threshold may relate to a pressure of at least 2 kPa, more specifically at least 6 kPa and in particular at least 20 kPa. In some embodiments, the force threshold may relate to a force of between about 2 kPa to about 1000 kPa, more specifically between about 6 kPa Newton to about 200 kPa and in particular between about 20 kPa to about 60 kPa.

A medicament delivery device 206, such as an insulin delivery device, like an insulin pump, may be provided. The medicament delivery device 206 may have a control system for controlling delivery of a medicament to the user responsive, at least in part, to sensor data from the biological sensor 202. In some exemplary embodiments, the medicament delivery device 206 is an automated insulin delivery (AID) device that delivers insulin to a user at regular intervals based at least in part of glucose level readings from a CGM. An automated drug delivery (ADD) device may alternatively be used that delivers an alternate medicament or drug therapy. The medicament may be insulin for treating diabetes. The medicament may be glucagon for raising a user's glucose level. The medicament also may be a glucagon-like peptide (GLP)-1 receptor agonists for lowering glucose or slowing gastric emptying, thereby delaying spikes in glucose after a meal. The medicament may also be pramlintide. The medicament may be a co-formulation of insulin and GLP-1 or pramlintide. Alternatively, the medicament delivered by the medicament delivery device 206 may be a pain relief agent, a chemotherapy agent, an antibiotic, a blood thinning agent, a hormone, a blood pressure lowering agent, an antidepressant, an antipsychotic, a statin, an anticoagulant, an anticonvulsant, an antihistamine, an anti-inflammatory, a steroid, an immunosuppressive agent, an antianxiety agent, an antiviral agent, a nutritional supplement, a vitamin, or co-formulations of two or more of the above.

A management device 210 for managing the medicament delivery device 206 may be provided. The management device 210 may be a special purpose device, such as a dedicated personal diabetes manager (PDM) device. The management device 210 may be a programmed general-purpose device, such as any portable electronic device including, for example, a dedicated controller, such as a processor, a micro-controller, or the like. The management device 210 may be used to program or adjust operation of the medicament delivery device 206 and/or the sensors 202 and 204. The management device 210 may be any portable electronic device including, for example, a smartphone, a smartwatch, or a tablet. Via the management device 210, a user may view a history of biological sensor values and medicament delivery history and may issue commands to the medicament delivery device 206.

In some exemplary embodiments, a data logger 208 may be provided. The data logger 208 is shown in phantom form to indicate that it is optional and may not be used in all embodiments. The data logger 210 logs data from the biological sensor 202 and the compression sensor 204. The data logger may transmit the sensor data to the medicament delivery device 206 and/or the management device, as will be described below. The data logger 210 may be useful when the biological sensor 202 is a standalone device and is not co-located with the medicament delivery device 206.

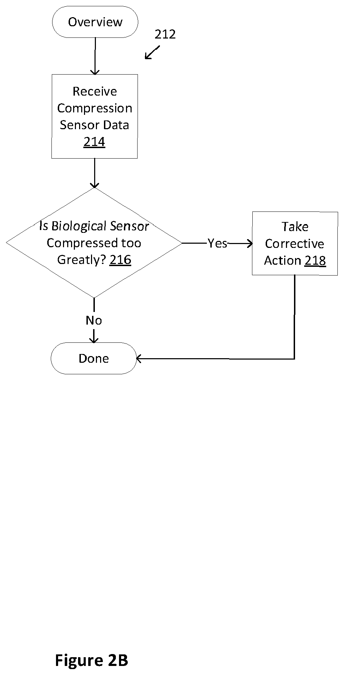

Figure 2B depicts a flowchart 212 of illustrative steps that may be performed in exemplary embodiments to sense and respond to compression of biological sensor 202, such as a glucose sensor that measures glucose levels of a user. At 214, compression sensor data is received by the medicament delivery device 206, the management device 210 or data logger 208 from compression sensor 204. Compression sensor 204 senses compression of the biological sensor 202, such as when a user lies on top of the biological sensor. At 216, compression sensor data is processed to determine if the biological sensor 202 is too greatly compressed (i.e., excessively compressed). The compression sensor data may specify an amount of pressure or force that is applied to the biological sensor 202. At 216, a pressure or force threshold may be established, and if the force or pressure threshold is exceeded, it can be concluded that the biological sensor 202 is excessively compressed. In still other instances, the compression sensor may act like a switch that only switches to a compressed state when enough force is applied to fully actuate a lever or other actuatable element. There also may be a time component in determining whether the biological sensor 202 is excessively compressed. Specifically, the compression or other measured force or pressure must last at least a minimum amount of time before the biological sensor 202 is deemed to be compressed too greatly (e.g., 5 minutes; 10 minutes; 15 minutes; 30 minutes; or another period of time). The minimum amount of time may also be referred to as threshold duration. In some embodiments, the threshold duration may be between about 10 second to about 60 minutes, more specifically between about 1 minute to 30 minutes and in particular between about 5 minutes to about 20 minutes.

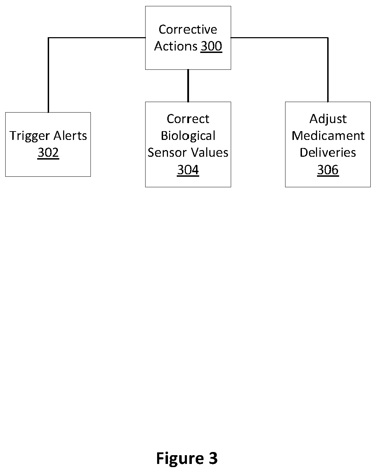

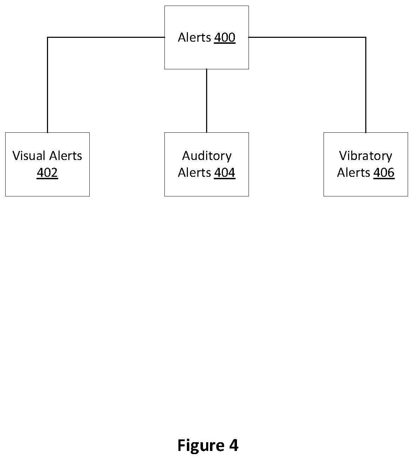

If it determined at 216 that the biological sensor 202 is compressed too greatly, corrective action may be taken at 218. Otherwise, no corrective action may be taken. Figure 3 depicts examples of possible corrective actions 300. A first variety of corrective action 300 is to trigger alerts 300 to alert the user to the compression and possibly to suggest user actions that may remedy the compression. Figure 4 depicts illustrative types of alerts 400. The alerts 400 may be visual alerts 402, such as textual and/or graphical content displayed on a display or via a light or LED of medicament delivery device 206, or on a display or user interface of the management device 210 for the medicament delivery device 206 or for the biological sensor 202. The alerts may warn the user of the compression and possibly suggest corrective action. The alerts 400 may include auditory alerts 404, such as beeps, sirens or spoken messages. The alerts may also include vibratory alerts 406, that may be generated using, for example, a vibrating alert motor or a linear resonant actuator. In some instances, combinations of two or more of the visual alerts 402, auditory alerts 404 and vibratory alerts 406 may be used.

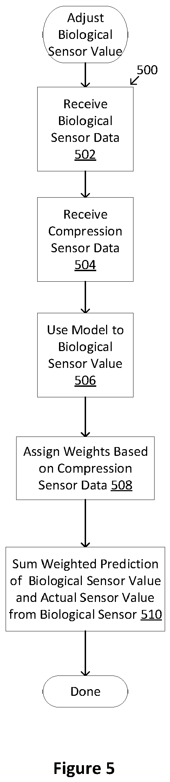

In addition to the triggering of alerts 302, the corrective action may include correcting the biological sensor data values 304 to account for the artificial change brought about by the compression of the biological sensor. One approach to correcting the biological sensor data values is depicted in the flowchart 500 of Figure 5. At 502, the biological sensor data is received. For example, glucose level data may be received from a glucose monitor, like a CGM. At 504, compression sensor data is received. At 506, a model is used to predict what the sensed data value would have been but for the excessive compression. The model may simply identify when an excessive compression occurs and predict what the expected sensed data values should have been while the biological sensor is excessively compressed but for the excessive compression. In other words, the model may generate predicted biological sensor data based on the biological sensor data sensed by the biological data when identifying an excessive compression (e.g. when the compression sensor data exceeds the compression threshold). In particular, in some embodiments, the model may generate the predicted biological sensor data additionally based on the compression sensor data from the compression sensor. The model may use interpolation on the plot of biological sensor data and other techniques to predict the expected biological sensor values. At 508, for each affected biological sensor value, weights are assigned to the biological sensor data value and to the corresponding expected biological sensor value produced by the model based on the compression sensor data (indicating the magnitude of the compression). At 510, the weighted prediction biological sensor data value and the actual biological sensor value are summed to produce a compensated biological sensor data value. This summing may be expressed as:

$$BD_{true} = a * BD_{raw} + b * BD_{pred}$$

where $BD_{true}$ is the compensated biological sensor value (e.g. true blood glucose value), $BD_{raw}$ is the biological sensor value from the biological sensor 202, $BD_{pred}$ is the predicted biological sensor value from the model, and $a$ and $b$ are the weight coefficients with values between 1 and 0 assigned based on the compression sensor data. Accordingly, in some embodiment correcting the biological sensor data values 304, in particular the glucose level data received from a glucose monitor, to account for the artificial change brought about by the compression of the biological sensor comprises calculating a weighted actual biological sensor value by multiplying a biological sensor value from the biological sensor with a weighting factor a. Correcting the biological sensor data values 304 further comprises calculating a weighted actual biological sensor value by multiplying a biological sensor value from the biological sensor with a weighting factor a and correcting the biological sensor additionally comprises calculating a weighted predicted biological sensor by multiplying the predicted biological sensor value from the prediction model with a weighting factor b, wherein a and b are calculated from the compression sensor data. Correcting the biological sensor data values 304 further comprises calculating a compensated biological sensor data value as the sum of the weighted actual biological sensor value and the weighted predicted biological sensor value. In some embodiments, the ratio of b to a increases with increasing compression indicated by the compression sensor data. In some embodiments, b may increase from 0 to 1 with increasing compression indicated by the compression sensor data and a may decrease from 1 to 0 with increasing compression indicated by the compression data. In some embodiments, b may be 0 and a may be 1 when compression sensor data indicated no compression or a compression below the compression threshold, and b may be 1 and a may be 0 at a second compression threshold. The weights may be based on empirical data. These weights capture the belief in the prediction model vs. the measured sensor data. As the compression forces increase the value of 'a' will be depreciated relative to the value of 'b'. The coefficients a and b can be mapped to the compression forces empirically. They may also be obtained experimentally or obtained by introducing a compression, measuring the compression, and then noting the raw sensor value. The true/predicted sensor value is known prior to the compression. Thereby estimates of coefficients a and b may be mapped to the measured compressive force to get a true estimate of blood glucose value.

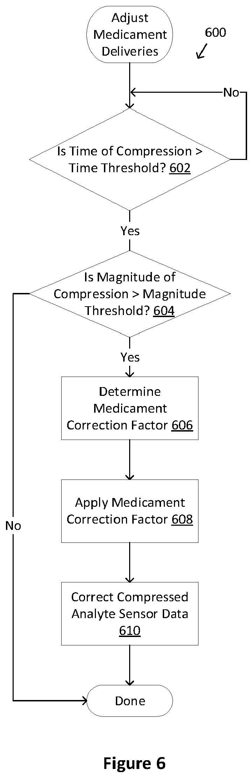

Another corrective action 300 is to adjust medicament deliveries 306 to account for the compression. Figure 6 depicts a flowchart 600 of illustrative steps that may be performed in exemplary embodiments to adjust the medicament deliveries to account for the compression. At 602, a check is made to see if the time of compression (i.e., the time period over which the biological sensor has remained compressed) exceeds a duration threshold. For example, with a glucose monitor, the check may be whether the glucose monitor has remained compressed for more than 5 minutes. The reason this check is made is that shorter compressions may not have much effect on the medicament delivery by the medicament delivery device 206. If not, the process continues to wait until sufficient time or a minimum threshold of compression has been reached. If so, at 604, a check is made whether the time of compression exceeds a magnitude threshold for compression. The notion of this check is that small magnitude compressions may not have any significant effect on the biological sensor 202. If not, the process finishes. If so, at 606, a medicament correction factor is determined based on the magnitude and duration of the compression. The medicament correction factor determines how much medicament would have been delivered but for the effect of the compression.

At 608, the medicament correction factor may then be applied to deliver any compensatory medicament or may adjust the delivery schedule of the medicament to account for the effect of the compression. At 610, the biological sensor data history may be corrected to make the history correct so that the control system for the medicament delivery device 206 makes decisions based on correct historical data. In other embodiments when compression is noted the medicament delivery device may deliver a nominal or basal rate of delivery. In particular, as the compression is relieved it may present as rising blood glucose and may trigger an automatic bolus delivery. By detecting the rapid change in glucose in conjunction with a decrease in detected compression, the system may determine that this rise is not a true rise in blood glucose but rather an increase due to the release of the compression, and the system may refrain from triggering an automatic bolus or increased basal rate. In some embodiments, the corrective action does not include delivering a medicament to the user, in particular in some embodiments, the corrective action does not include delivering insulin to the user.

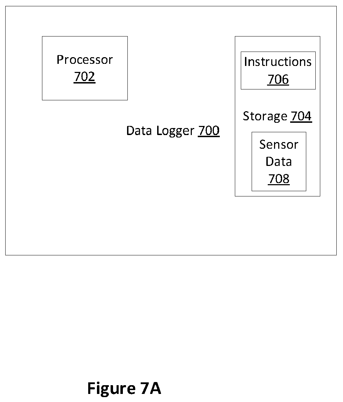

As was mentioned above, in some exemplary embodiments, a data logger may be used. Figure 7A depicts components of an illustrative data logger 700. The data logger 700 may include a processor 702. The processor 702 may be a central processing unit (CPU), a graphics processing unit (GPU), an application specific integrated circuit (ASIC), a field programmable gate array (FPGA) or another type of microprocessor. The data logger 700 may include storage 704. The storage may be a memory or may be a database in a cloud computing environment. The storage 704 may comprise one or more memory or storage devices. For instance, the storage 704 may include types of random access memory (RAM), read only memory (ROM), solid state memory, magnetic disk storage, optical disk storage and generally may include non-transitory computer-readable storage media. The storage 704 may store computer programming instructions 706 for execution by the processor for performing functionality of the data logger 700 described herein.

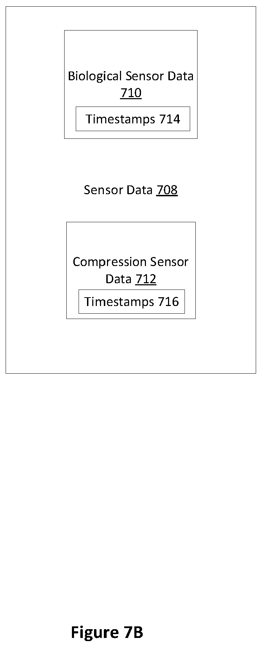

The storage 704 also may store sensor data 708. As shown in the Figure 7B, the sensor data 708 may include biological sensor data 710 from the biological sensor 202. The sensor data 708 also may include compression sensor data 712 from the compression sensor 204. The biological sensor data 710 may include timestamps 714. For example, where the biological sensor 202 is a glucose sensor, the biological sensor data may include glucose values from the glucose sensor 202 where each glucose value has an associated timestamp 714 indicative of when the glucose value was logged or sensed. Similarly, the compression sensor data 712 includes associated timestamps 716.

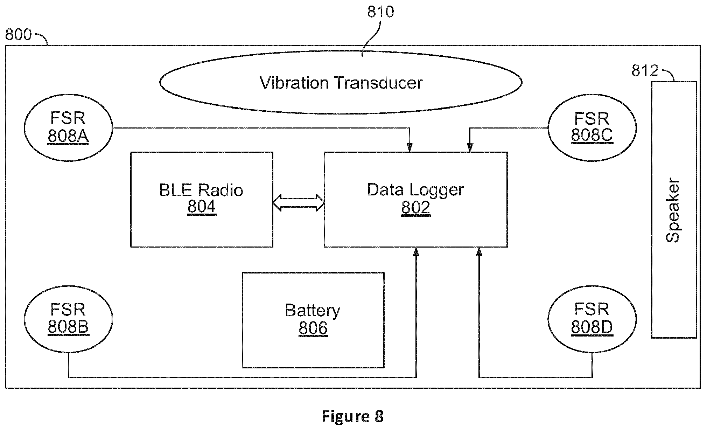

The data logger may be part of a data logger and wireless communication subsystem 800 as depicted in Figure 8. The data logger 802 may communicate with a wireless radio component 804 that facilitates wireless communications via a wireless protocol, such as Bluetooth Low Energy (BLE). The wireless component may include a wireless transceiver for receiving and transmitting wireless communications. A battery 806 may be provided to power the subsystem 800. Force sensitive resistors 808A, 808B, 808C and 808D may be provided to act as the compression sensor 204. Multiple force sensitive resistors 808A, 808B, 808C and 808D are provided to provide coverage across the biological sensor. A vibration transducer 810 may be provided to provide vibratory alerts. A speaker 812 may be provided to deliver auditory output.

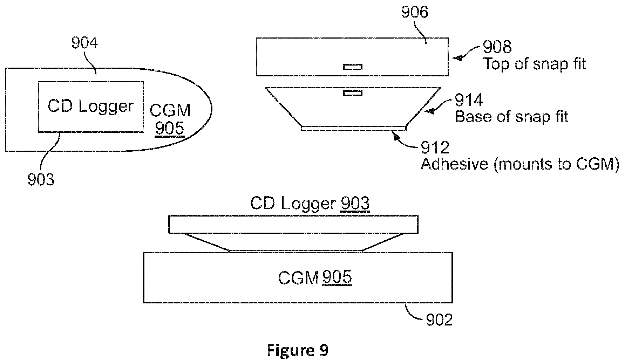

Figure 9 depicts three different views 902, 904 and 906 of the data logger. View 902 shows the data logger 903 secured to a top surface of the biological sensor 905, which in the depicted example is a CGM. The biological sensor is designed to be secured to the skin of the user, such as through an adhesive pad. View 904 depicts a top view of the data logger 903 and the biological sensor 905. As can be seen in view 904, the biological sensor 905 has a larger footprint than the data logger 903. View 906 depicts the data logger 903 in partially exploded form. The data logger 903 may contain a top portion 908 that may snap fit with a base portion 910. An adhesive layer 912 is secured to the bottom face of the base portion, such as via a snap fit. The adhesive on the adhesive layer 912 secures the data logger to the biological sensor 905 as shown in views 902 and 904.

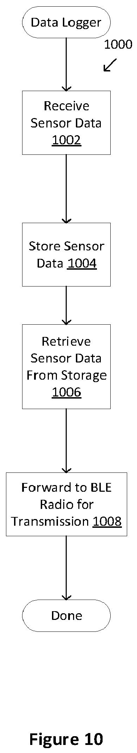

Figure 10 depicts a flowchart 1000 of illustrative steps that may be performed by the data logger 700 in exemplary embodiments. At 1002, the data logger 700 receives sensor data 708, which includes biological sensor data 710 with timestamps 714 and the compression sensor data 712 with timestamps 716. At 1004, the data logger 700 stores the received sensor data 710 in the storage 704. Subsequently, the sensor data 710 is retrieved from the storage 704 at 1006 and then at 1008 sent to the wireless radio component 804 for transmission, such as to the medicament delivery device 206 or the management device 210. The control system of the medicament delivery device 206 may use the sensor data 710 as input. The management device 210 may store the sensor data 710 and use the stored sensor data 710 in providing history information to the user, determining insights on performance and use of the medicament delivery device, etc.

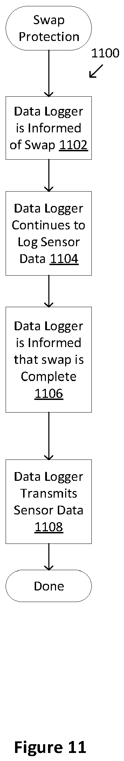

The data logger 700 may prevent loss of data when a medicament delivery device 206 is swapped out for replacement by a new medicament delivery device. For example, some insulin pumps are designed for 3 days of use and are swapped out for a new insulin pump after 3 days. As mentioned above, during the swapping, biological sensor data may be lost. Figure 11 depicts a flowchart 1100 of illustrative steps that may be performed in exemplary embodiments to avoid loss of sensor data during the swapping. At 1102, the data logger 700 is informed of the swap. At 1104, the data logger 700 continues to log the sensor data 710 but does not transmit to the medicament delivery device 206 or the management device 210 yet. At 1106, the data logger 700 is informed that the swap is complete. At 1108, the data logger swaps the sensor data 710 to the medicament delivery device 206 and perhaps to the management device 210.

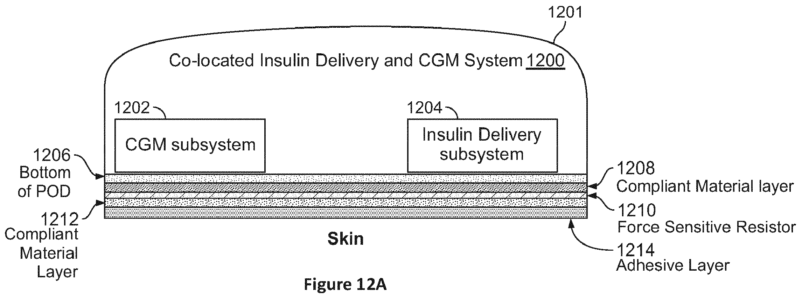

In other exemplary embodiments, the data logger is not used. In one such exemplary embodiment the data logger and the medicament delivery device are co-located and within a common or adjoined housing to form a single device. Figure 12A depicts an example of a co-located insulin and CGM system 1200. A CGM subsystem 1202 is co-located with an insulin delivery subsystem in a common housing or housings 1201. The bottom of the co-located insulin and CGM system 1200 may have a compliant material layer 1208 secured to it. The term "compliant material layer" as described herein, may relate to a material layer that deforms when subjected to a force, in particular wherein the material layer deforms elastically. Additionally or alternatively, the term "compliant material layer" may relate to a material aye with a Young's modulus of up to 5 GPa, more specifically with a Young's modulus between about 0.01 GPa to about 5 GPa. In some embodiments, the compliant material may exhibit a Young's modulus between about 0.1 GPa to about 3.5 GPa, more specifically between about 0.2 GPa to about 2 GPa. A force sensitive resistor 1210 may be positioned between the compliant material layer 1208 and another compliant material layer 1212. An adhesive layer 1215 may be secured to the compliant material layer 1212. An adhesive layer 1214 contains an adhesive for securing the co-located insulin delivery and CGM system 1200 to the skin of a user. The compliant material layers 1208 and 1212 are present so that the pressure put on the co-located insulin delivery and CGM system 1200 is transmitted to the force sensitive resistor that is acting as the compression sensor 204. In some embodiments, only a single compliant material layer is used, or alternatively, the adhesive layer acts as a compliant material layer. The force sensitive resistor 1212 may be sandwiched between the compliant material layers 1208 and 1212, secured to one the compliant material layers 1208 and 1212, formed in one of compliant material layers 1208 and 1212 or secured directly to the bottom of the co-located insulin delivery and CGM system 1200.

The CGM subsystem 1202 and the insulin delivery subsystem 1202 act like the biological sensor 202 and medicament delivery device 206 described above. Sensor data from the force sensitive resistor 1210 and sensor data from the CGM subsystem 1202 are passed to the insulin delivery subsystem. The sensor data is processed, and the steps of Figure 2B may be performed to detect and take corrective action regarding compression of the CGM subsystem 1202. The CGM subsystem and the force sensitive resistor may have electrical connections with the insulin delivery subsystem for communication rather than wireless connections and a data logger.

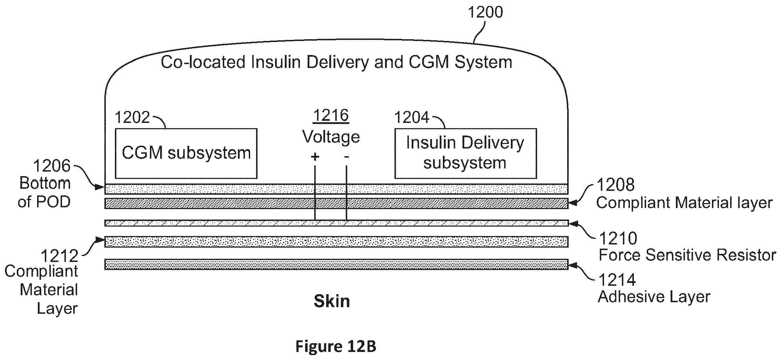

In some exemplary embodiments, as shown in Figure 12B, the co-located insulin delivery and CGM system may provide a voltage supply 1316 to force sensitive resistor. Otherwise, the components 1201, 1202, 1204, 1206, 1208, 1210, 1212 and 1214 may remain the same as shown in Figure 12A and discussed above.

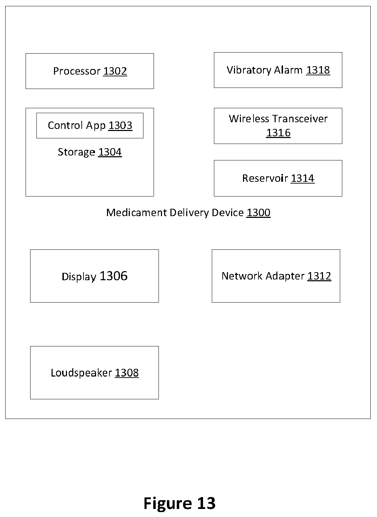

Figure 13 depicts components of an illustrative medicament delivery device in more detail than Figure 2A. The medicament delivery device 1300 may contain a processor 1302, such as a CPU, a GPU, an ASIC, a FPGA or the like. The processor 1302 may run a control application 1303 formed of computer programming instructions for controlling operation of the medicament delivery device 1300. The control application 1303 may be stored in a storage 1304. The storage 1304 may contain non-transitory computer-readable storage media for storing computer programming instructions and data. The storage may include memory and storage devices, such as RAM devices, ROM devices, solid state memory devices, optical disk storage device, magnetic storage devices and the like.

The medicament delivery device 1300 may contain a display 1306 for showing textual and/or graphical or video content. The display 1306 may display alerts as discussed above. The medicament delivery device 1300 may contain a loudspeaker 1308 for outputting audio content, such as auditory alerts. The medicament delivery device 1300 may include a vibratory alarm 1318 for providing a vibratory alert as discussed above. A wireless transceiver 1316 may be provided for enabling wireless communications. A reservoir 1314 for holding medicament may be provided. A network adapter 1312 may be provided for connecting to a network.

In another embodiment, patterns in the CGM may be learnt by the processor 1302 of the medicament delivery device 1300 to identify patterns as those of false lows, in particular false lows due to compression of the CGM. For example, the processor 1302 may have a look ahead prediction of the glucose value. If the look ahead prediction is elevated compared to the measured value, and a recent downward glucose trend has been observed, the processor 1302 may determine that this depression in glucose value is because of compression, in particular due to compression of the CGM. In particular, if the processor 1302 starts to detect an upward trend again in glucose values and the gap between the predicted and measured values is now decreasing, the system may determine that this is due to compression relief. The processor 1302 may deliver basal insulin on the downward trending CGM values and not trigger a bolus insulin when the CGM values starts to rise.

While exemplary embodiments have been described herein, various changes in form and detail may be made without departing from the intended scope of the appended claims. It should be understood, that the embodiments of the present disclosure may be suitably combined.

**Embodiments**

Although the present invention is defined in the attached claims, it should be understood that the present invention can also (alternatively) be defined in accordance with the following embodiments:

1. An electronic device secured to a user, comprising:

> a medicament delivery unit for delivering medicament to the user;
> a biological sensor for sensing biological data of the user and generating biological sensor data indicative of the sensed biological data;
> a compression sensor for sensing compression of the biological sensor and/or biological tissue of the user and generating compression sensor data; and
> a processor configured for receiving the compression sensor data from the compression sensor and taking corrective action when the compression sensor data from the compression sensor indicates excessive compression of the biological sensor and/or biological tissue of the user.

2. The electronic device of embodiment 1, wherein the corrective action includes triggering an alarm that indicates to the user that there is the excessive compression of the biological sensor and/or biological tissue of the user.

3. The electronic device of embodiment 2, wherein the alarm is part of the electronic device and the alarm includes at least one of a vibratory alarm, an audio alarm or a visual alarm.

4. The electronic device of embodiment 1, wherein the corrective action includes one of modifying an amount of medicament delivered to the user by the medicament delivery unit to compensate for the excessive compression or adjusting the biological sensor data from the biological sensor to compensate for the excessive compression.

5. The electronic device of embodiment 1, further comprising one or more housings for encasing the medicament delivery unit and the biological sensor, wherein the one or more housings is a single housing, wherein the electronic device includes a compliant layer to which the compression sensor is secured or positioned against, and wherein the compliant layer is secured to the housing.

6. The electronic device of embodiment 5, further comprising an additional compliant layer positioned so that the compression sensor is situated between the compliant layer and the additional compliant layer.

7. The electronic device of embodiment 1, further comprising an adhesive layer having an adhesive for securing the electronic device to the user.

8. A glucose monitor, comprising:

a glucose sensor for sensing a glucose level of a user and generating glucose level readings indicative of the sensed glucose level;
a compression sensor for sensing amounts of compression of the glucose sensor and generating compression sensor readings indicative of the sensed amounts of compression;
a memory for storing the glucose level readings and the compression sensor readings;
a wireless transmitter for sending the glucose level readings and the compression sensor readings to an external electronic device; and
a processor configured for storing the glucose level readings and the compression sensor readings in the memory and forwarding the glucose level readings from the memory to the wireless transmitter for transmission to the external electronic device.

9. The glucose monitor of embodiment 8, wherein the processor is further configured to forward at least some of the compression sensor readings from the memory to the wireless transmitter for transmission to the external electronic device.

10. The glucose monitor of embodiment 8, wherein the external electronic device is one of an insulin delivery device or a management device for the insulin delivery device or the glucose monitor, and wherein the processor is further configured to cause the wireless transmitter to transmit at least some of the glucose level readings and compression sensor readings from the memory to the insulin delivery device or the management device.

11. The glucose monitor of embodiment 8, further comprising one or more alarms comprising an auditory alarm and/or a vibratory alarm for warning the user of excessive compression of the glucose monitor and/or biological tissue of the user.

12. The glucose monitor of embodiment 11, the processor being further configured to receive an indication that there is excessive compression of the glucose monitor and/or the biological tissue of the user from the external device and to trigger the one or more alarms responsive to receiving the indication.

13. A method performed by a processor of an electronic device, comprising:

receiving sensor data from a compression sensor of a glucose monitor for a user;
processing the sensor data from the compression sensor with the processor to determine that the glucose monitor and/or biological tissue of the user is being compressed; and
with the processor, triggering corrective action responsive to the determining that the glucose monitor and/or biological tissue of the user is being compressed.

14. The method of embodiment 13, wherein the compression sensor is an electronic sensor that senses pressure or magnitude of force.

15. The method of embodiment 13, wherein the compression sensor is a mechanical sensor.

16. The method of embodiment 13, wherein the compression sensor is secured to a bottom surface of the glucose monitor that faces the user.

17. The method of embodiment 13, wherein the corrective action includes initiating one or more alarms to alert the user of the glucose monitor and/or the biological tissue of the user is being compressed.

18. The method of embodiment 13, wherein the corrective action comprises adjusting glucose readings from the glucose monitor to compensate for the compression.

19. The method of embodiment 13, wherein the corrective action is sending data to an insulin delivery device or a management device of the insulin delivery device indicative of the excessive compression of the glucose monitor and/or the biological tissue of the user.

20. The method of embodiment 13, wherein the electronic device is an insulin delivery device attached to the user or a management device for the insulin delivery device or the glucose monitor.

**Claims**

1. An electronic device configured to be secured to a user, comprising:

a medicament delivery unit for delivering medicament to the user;
a biological sensor for sensing biological data of the user and generating biological sensor data indicative of the sensed biological data;
a compression sensor for sensing compression of the biological sensor and/or biological tissue of the user and generating compression sensor data; and

a processor configured for receiving the compression sensor data from the compression sensor and taking corrective action when the compression sensor data from the compression sensor indicates excessive compression of the biological sensor and/or biological tissue of the user.

2. The electronic device of claim 1, wherein the compression sensor indicates excessive compression, when the compression sensor data exceeds a compression threshold.

3. The electronic device of claim 1 or 2, wherein the corrective action includes triggering an alarm, more specifically wherein the corrective action includes trigger an alarm that indicates to the user that there is the excessive compression of the biological sensor and/or biological tissue of the user.

4. The electronic device of claim 3, wherein the alarm is part of the electronic device and the alarm includes at least one of a vibratory alarm, an audio alarm or a visual alarm.

5. The electronic device of any one of claims 1 to 4, wherein the corrective action includes at least one of modifying an amount of medicament delivered to the user by the medicament delivery unit to compensate for the excessive compression or adjusting the biological sensor data from the biological sensor to compensate for the excessive compression.

6. The electronic device of any one of claims 1 to 5, wherein the medicament delivery unit and the biological sensor are encased by a single housing, wherein the electronic device includes a compliant layer to which the compression sensor is secured or positioned against, and wherein the compliant layer is secured to the single housing.

7. The electronic device of claim 6, further comprising an additional compliant layer positioned so that the compression sensor is situated between the compliant layer and the additional compliant layer.

8. A glucose monitor, comprising:

a glucose sensor for sensing a glucose level of a user and generating glucose level readings indicative of the sensed glucose level;
a compression sensor for sensing amounts of compression of the glucose sensor and generating compression sensor readings indicative of the sensed amounts of compression;

a memory for storing the glucose level readings and the compression sensor readings;
a wireless transmitter for sending the glucose level readings and the compression sensor readings to an external electronic device; and
a processor configured for storing the glucose level readings and the compression sensor readings in the memory and forwarding the glucose level readings from the memory to the wireless transmitter for transmission to the external electronic device.

9. The glucose monitor of claim 8, wherein the processor is further configured to forward at least some of the compression sensor readings from the memory to the wireless transmitter for transmission to the external electronic device.

10. The glucose monitor of claims 8 or 9, wherein the external electronic device is one of an insulin delivery device or a management device for the insulin delivery device or the glucose monitor, and wherein the processor is further configured to cause the wireless transmitter to transmit at least some of the glucose level readings and compression sensor readings from the memory to the insulin delivery device or the management device.

11. The glucose monitor of any one of claims 8 to 10, further comprising one or more alarms comprising an auditory alarm and/or a vibratory alarm for warning the user of excessive compression of the glucose monitor and/or biological tissue of the user.

12. The glucose monitor of claim 11, the processor being further configured to receive an indication that there is excessive compression of the glucose monitor and/or the biological tissue of the user from the external device and to trigger the one or more alarms responsive to receiving the indication.

13. A method performed by a processor of an electronic device, comprising:

receiving sensor data from a compression sensor of a glucose monitor for a user;
processing the sensor data from the compression sensor with the processor to determine that the glucose monitor and/or biological tissue of the user is being compressed; and
with the processor, triggering corrective action responsive to the determining excessive compression of the glucose monitor and/or biological tissue of the user.

14. The method of claim 13, wherein the compression sensor is an electronic sensor that senses pressure or magnitude of force;

or wherein the compression sensor is a mechanical sensor.

15. The method of claim 13 or 14, wherein the compression sensor is secured to a bottom surface of the glucose monitor configured to face the user.

16. The method of any one of claims 13 to 15, wherein the corrective action includes initiating one or more alarms to alert the user of the glucose monitor and/or the biological tissue of the user is being compressed; and/or

   wherein the corrective action comprises adjusting glucose readings from the glucose monitor to compensate for the compression; and/or wherein the corrective action is sending data to an insulin delivery device or a management device of the insulin delivery device indicative of the excessive compression of the glucose monitor and/or the biological tissue of the user.

17. The method of any one of claim 13 to 16, wherein the electronic device is an insulin delivery device configured to be attached to the user or a management device for the insulin delivery device or the glucose monitor.

Figure 1

200

Biological Sensor 202

Compression Sensor 204

Data Logger 208

Medicament Delivery Device 206

Management Device 210

**Figure 2A**

```
        ┌──────────┐
        │ Overview │        212
        └────┬─────┘         ↙
             │
             ▼
      ┌──────────────┐
      │   Receive    │
      │ Compression  │
      │ Sensor Data  │
      │     214      │
      └──────┬───────┘
             │
             ▼
          ╱╲╲
    Is Biological Sensor          ┌──────────────┐
    Compressed too  ──── Yes ───▶ │    Take      │
    Greatly? 216                  │  Corrective  │
          ╲╱                      │  Action 218  │
           │                      └──────┬───────┘
           No                            │
           │                             │
           ▼                             │
      ┌────────┐                         │
      │  Done  │ ◀───────────────────────┘
      └────────┘
```

**Figure 2B**

EP 4 268 719 A1

```
                    ┌─────────────┐
                    │  Corrective │
            ┌───────│ Actions 300 │───────┐
            │       └──────┬──────┘       │
            │              │              │
     ┌──────┴──────┐ ┌─────┴──────┐ ┌─────┴──────┐
     │             │ │  Correct   │ │   Adjust   │
     │Trigger Alerts│ │ Biological │ │ Medicament │
     │     302     │ │Sensor Values│ │ Deliveries │
     │             │ │    304     │ │    306     │
     └─────────────┘ └────────────┘ └────────────┘
```

**Figure 3**

Visual Alerts
402

Alerts 400

Auditory
Alerts 404

Vibratory
Alerts 406

**Figure 4**

Adjust
Biological
Sensor Value

500

Receive
Biological
Sensor Data
502

Receive
Compression
Sensor Data
504

Use Model to
Biological
Sensor Value
506

Assign Weights Based
on Compression
Sensor Data 508

Sum Weighted Prediction
of Biological Sensor Value
and Actual Sensor Value
from Biological Sensor 510

Done

**Figure 5**

Figure 6

Processor
702

Instructions
706

Storage 704

Sensor
Data
708

Data Logger 700

**Figure 7A**

Biological Sensor Data
710

Timestamps 714

Sensor Data 708

Compression Sensor
Data 712

Timestamps 716

**Figure 7B**

Figure 8

**Figure 9**

Data Logger

1000

Receive
Sensor Data
1002

Store Sensor
Data 1004

Retrieve
Sensor Data
From Storage
1006

Forward to BLE
Radio for
Transmission 1008

Done

**Figure 10**

**Figure 11**

Co-located Insulin Delivery and CGM System 1200

1201

1202
CGM subsystem

1204
Insulin Delivery subsystem

1206
Bottom of POD

1208
Compliant Material layer

1212
Compliant Material Layer

1210
Force Sensitive Resistor

1214
Adhesive Layer

Skin

**Figure 12A**

EP 4 268 719 A1

Figure 12B

Medicament Delivery Device 1300

Processor 1302

Control App 1303

Storage 1304

Vibratory Alarm 1318

Wireless Transceiver 1316

Reservoir 1314

Network Adapter 1312

Display 1306

Loudspeaker 1308

**Figure 13**

Europäisches
Patentamt

European
Patent Office

Office européen
des brevets

# EUROPEAN SEARCH REPORT

**Application Number**

EP 23 17 0041

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2021/228114 A1 (REBEC MIHAILO V [US] ET AL) 29 July 2021 (2021-07-29) * paragraphs [0042], [0099] – [0103], [0114] – [0118], [0124] – [0131], [0143], [0152] – [0155], [0172] – [0180]; claims; figures * | 1–17 | INV. A61B5/145 A61B5/00 A61M5/172 |
| X | US 2012/078071 A1 (BOHM SEBASTIAN [US] ET AL) 29 March 2012 (2012-03-29) * paragraphs [0107], [0115] – [0146], [0206], [0219], [0241], [0274] – [0288], [0308] – [0316]; claims; figures * | 1–17 | |
| X | US 2009/312615 A1 (CADUFF ANDREAS [CH] ET AL) 17 December 2009 (2009-12-17) * paragraphs [0030] – [0047], [0100] – [0112], [0130] – [0133]; claims; figures * | 8–17 1–7 | |
| A | WO 2022/006063 A1 (ENABLE INJECTIONS INC [US]) 6 January 2022 (2022-01-06) * paragraphs [0142], [0235], [0249] – [0255], [0266] – [0267], [0269], [0280] – [0281], [0340] * | 1–17 | **TECHNICAL FIELDS SEARCHED (IPC)** A61B A61M |

The present search report has been drawn up for all claims

| Place of search | Date of completion of the search | Examiner |
|---|---|---|
| The Hague | 25 September 2023 | Crisan, Carmen-Clara |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 23 17 0041

This annex lists the patent family members relating to the patent documents cited in the above-mentioned European search report.
The members are as contained in the European Patent Office EDP file on
The European Patent Office is in no way liable for these particulars which are merely given for the purpose of information.

25-09-2023

| Patent document cited in search report | | | Publication date | Patent family member(s) | | | Publication date |
|---|---|---|---|---|---|---|---|
| US 2021228114 | A1 | | 29-07-2021 | BR | 112022014591 | A2 | 27-09-2022 |
| | | | | CA | 3165705 | A1 | 29-07-2021 |
| | | | | CN | 115397313 | A | 25-11-2022 |
| | | | | EP | 4093268 | A1 | 30-11-2022 |
| | | | | US | 2021228114 | A1 | 29-07-2021 |
| | | | | WO | 2021151002 | A1 | 29-07-2021 |
| US 2012078071 | A1 | | 29-03-2012 | DK | 2621339 | T3 | 24-02-2020 |
| | | | | EP | 2621339 | A2 | 07-08-2013 |
| | | | | EP | 3632308 | A1 | 08-04-2020 |
| | | | | US | 2012078071 | A1 | 29-03-2012 |
| | | | | US | 2019167169 | A1 | 06-06-2019 |
| | | | | US | 2019350502 | A1 | 21-11-2019 |
| | | | | US | 2021251531 | A1 | 19-08-2021 |
| | | | | WO | 2012050926 | A2 | 19-04-2012 |
| US 2009312615 | A1 | | 17-12-2009 | EP | 1954175 | A1 | 13-08-2008 |
| | | | | EP | 2329764 | A2 | 08-06-2011 |
| | | | | JP | 4947440 | B2 | 06-06-2012 |
| | | | | JP | 2009514619 | A | 09-04-2009 |
| | | | | US | 2009312615 | A1 | 17-12-2009 |
| | | | | WO | 2007053963 | A1 | 18-05-2007 |
| WO 2022006063 | A1 | | 06-01-2022 | AU | 2021299214 | A1 | 23-02-2023 |
| | | | | BR | 112022027121 | A2 | 07-03-2023 |
| | | | | CA | 3183501 | A1 | 06-01-2022 |
| | | | | CN | 116234591 | A | 06-06-2023 |
| | | | | EP | 4175697 | A1 | 10-05-2023 |
| | | | | JP | 2023532459 | A | 28-07-2023 |
| | | | | US | 2023293108 | A1 | 21-09-2023 |
| | | | | WO | 2022006063 | A1 | 06-01-2022 |

EPO FORM P0459

For more details about this annex : see Official Journal of the European Patent Office, No. 12/82